# FASCICULE DE BREVET EUROPEEN

(11) **EP 0 588 676 B1**
(45) Date de publication et mention de la délivrance du brevet: **17.07.1996**
(21) Numéro de dépôt: 93402086.8
(22) Date de dépôt: 24.08.1993
(51) Int. Cl.: C07C 17/38, C07C 19/08, C01B 7/19

(54) **Procédé de séparation du fluorure d'hydrogène de ses mélanges avec le 1,1,1-Trifluoro-2-Chloroéthane**
Verfahren zur Abtrennung von Fluorwasserstoff aus seinen Gemischen mit 1,1,1-Trifluor-2-chlorethan
Process for the separation of hydrogen fluoride from its mixtures with 1,1,1-trifluoro-2-chloroethane

(30) Priorité: 10.09.1992 FR 9210811
(43) Date de publication de la demande: 23.03.1994
(73) Titulaire: ELF ATOCHEM S.A., F-92800 Puteaux (FR)
(72) Inventeur: Galland, Jean-Michel, F-69390 Vernaison (FR); Perdriau, René, F-69004 Lyon (FR); Rouzies, Dominique, F-69001 Lyon (FR)
(74) Mandataire: Leboulenger, Jean

(56) Documents cités:
- EP-A- 0 353 970
- EP-A- 0 509 885
- EP-A- 0 542 290
- FR-A- 2 414 475

## Description

L'invention concerne la séparation du fluorure d'hydrogène (HF) de ses mélanges avec le 1,1,1-rifluoro-2-chloroéthane (F133a) qui est un important intermédiaire de synthèse, utilisable notamment pour la fabrication du 1,1,1,2-tétrafluoroéthane (F134a).

Le procédé selon l'invention s'applique plus particulièrement à la séparation de l'HF non transformé contenu dans les mélanges provenant de la fabrication du F133a par fluoration du trichloréthylène ou du tétrachloroéthane symétrique ou asymétrique. Pour des raisons économiques, il est nécessaire de récupérer l'HF sous forme anhydre afin de pouvoir le recycler au réacteur de fluoration.

Diverses techniques pour effectuer la séparation d'HF et d'hydrocarbures chlorofluorés ont déjà été décrites. On peut citer par exemple :
- le brevet US 2 640 086 qui concerne la séparation de l'HF et du chlorodifluorométhane et utilise du chloroforme pour favoriser la décantation en deux phases, une phase riche en HF et une phase pauvre en HF ;
- le brevet US 3 873 629 relatif à un procédé continu pour la séparation d'HF et de chlorodifluorométhane et consistant à mettre en contact à contre-courant le mélange gazeux des deux constituants avec de l'acide sulfurique ;
- le brevet US 3 976 447 qui propose une séparation de l'HF d'effluents gazeux par absorption-désorption sur des particules de chlorure de calcium, baryum ou strontium ;
- le brevet US 4 209 470 qui décrit un procédé de séparation de l'HF de ses mélanges avec le 1-chloro-1,1-difluoroéthane dans lequel, pour améliorer la décantation, on ajoute un liquide auxiliaire constitué totalement-ou majoritairement par du 1,1-dichloro-1-fluoroéthane ;
- la demande de brevet EP 0 353 970 relative à la séparation d'HF de ses mélanges avec le 2,2-dichloro-1,1,1-trifluoroéthane et/ou le 2-chloro-1,1,1,2-tétrafluoroéthane par décantation et distillation.

Dans le cas des mélanges d'HF et de F133a, une simple distillation ne permet pas de les séparer car l'HF et le F133a forment un azéotrope plus volatil que l'HF ou le F133a ; la teneur en HF de cet azéotrope est d'environ 60 % molaire (20 % en poids). A température ambiante et quelles que soient les concentrations en HF et F133a, les mélanges d'HF et de F133a ne décantent pas en deux phases.

On peut, par contre, obtenir une excellente séparation d'un mélange d'HF et de F133a à condition de le refroidir à une température inférieure à 0°C, de préférence comprise entre - 40°C et - 10°C. Il a également été trouvé que l'on peut encore améliorer cette séparation si la décantation de l'HF et du F133a est effectuée en présence de trichloroéthylène (TCE).

La présence de TCE dans le mélange de F133a et d'HF diminue sensiblement les solubilités de l'HF dans la phase organique et des organiques dans la phase HF ; la séparation du F133a et de l'HF s'effectue donc de manière plus efficace.

L'invention a donc pour objet un procédé de séparation d'HF et de F133a, caractérisé en ce qu'il comprend une étape dans laquelle le mélange HF-F133a est soumis à une décantation en présence de TCE à une température inférieure à 0°C pour obtenir une phase supérieure riche en HF et une phase inférieure organique pauvre en HF, le mélange soumis à la décantation contenant de 0,25 à 2,5 moles de TCE par mole de F133a.

La proportion de TCE dans le mélange soumis à décantation comprise entre 0,8 et 1,2 mole par mole de F133a. Le TCE peut éventuellement déjà être présent dans le mélange initial de F133a et d'HF (par exemple dans le cas où le mélange à traiter proviendrait d'une fabrication de F133a à partir de trichloroéthylène et d'HF) ou peut être rajouté au mélange HF-F133a (gazeux ou liquide) préalablement à la décantation. Le mélange HF-F133a-TCE est ensuite amené aux conditions de température et de pression choisies pour la décantation. Celle-ci est avantageusement effectuée à une température comprise entre -40 et -10°C, de préférence entre -25 et -15°C. La pression n'a pas d'influence notable sur la décantation ; on peut donc généralement opérer à une pression allant de 1 à 30 bars absolus, même si en pratique on préfère travailler à une pression allant de 1 à 15 bars absolus.

L'opération de décantation peut être réalisée en continu ou en discontinu et selon diverses techniques connues. Elle peut être avantageusement combinée avec d'autres opérations de séparation telles que des distillations en fonction de l'objectif de séparation recherché. Ainsi, si l'on désire obtenir le F133a quasi pur, l'HF quasi pur et le TCE quasi pur pour recyclage, la décantation peut être combinée aux opérations suivantes :
**a)** La phase inférieure organique pauvre en HF, obtenue par décantation, est distillée de manière à récupérer l'HF en tête d'une colonne à distiller, sous forme d'azéotrope HF-F133a qui est recyclé au décanteur, et à récupérer le TCE et le F133a en pied de colonne.
**b)** La phase supérieure riche en HF, obtenue par décantation est soumise à une distillation de manière à récupérer en tête d'une colonne à distiller le F133a sous forme d'azéotrope HF-F133a qui est recyclé au décanteur et à récupérer l'HF pratiquement pur en pied de colonne.
**c)** Le mélange de TCE et de F133a obtenu en pied de la colonne citée en a) est soumis à une distillation de manière à récupérer en tête le F133a purifié et en pied le TCE qui peut par exemple être recyclé à la décantation.

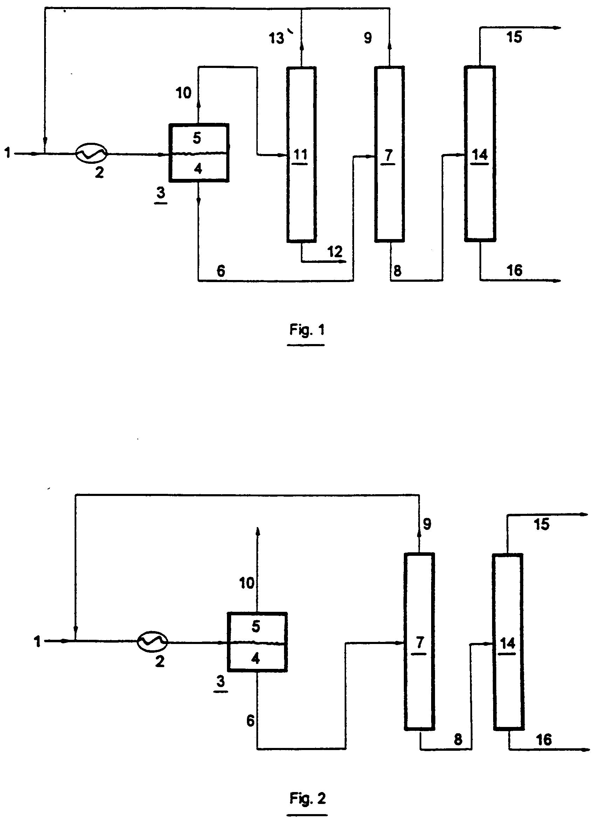

Le fonctionnement du procédé selon l'invention dans sa version complète sera mieux compris en se référant au schéma de la figure 1 annexée. Après ajout éventuel de TCE, le mélange à traiter est introduit par la conduite 1 pour être préalablement refroidi par l'intermédiaire de l'échangeur 2. Il est ensuite amené au décanteur 3 maintenu à une température inférieure à 0°C, de préférence comprise entre - 40°C et - 10°C. Par démixtion, on obtient alors dans le décanteur une phase inférieure organique 4, pauvre en HF, et une phase supérieure 5, riche en HF. La phase organique 4, issue du décanteur 3 alimente par 6 une colonne à distiller 7 au sommet de laquelle on sort un effluent 9 de composition azéotropique en HF et F133a ; cet effluent 9 est renvoyé au décanteur 3 en amont de l'échangeur 2 pour séparation en deux phases. En pied de la colonne 7, on récupère un flux 8 constitué d'un mélange de F133a et de TCE.

Le flux 8 est soumis à une distillation dans la colonne 14 permettant d'obtenir en tête par la ligne 15 le F133a pur et en pied par la ligne 16 le TCE qui est soit utilisé en l'état, soit recyclé en amont du refroidisseur 2 comme le flux 9.

La phase supérieure 5, riche en HF, est amenée par la conduite 10 à une colonne à distiller 11, en tête de laquelle on sort un effluent 13, de composition azéotropique en HF et F133a qui, comme l'effluent 9, est renvoyé au décanteur 3 en amont de l'échangeur 2, pour séparation en deux phases. En pied de la colonne 11, on récupère alors en 12 un HF pratiquement pur.

Le passage du décanteur aux colonnes à distiller et de celles-ci vers le décanteur se fait par l'intermédiaire de vannes de détente ou de pompes selon les pressions de fonctionnement du décanteur et des colonnes à distiller. La température des flux alimentant par 6 et 10 les colonnes à distiller peut être ajustée par l'intermédiaire d'échangeurs pour obtenir une distillation optimale.

La figure 2 illustre un autre mode de mise en oeuvre du procédé où la phase supérieure 5, riche en HF, sortant par la conduite 10, peut être recyclée telle quelle directement au réacteur de fluoration pour la production de F133a.

Le procédé selon l'invention convient pour traiter des mélanges contenant une quantité prépondérante d'HF et de F133a (% HF + % F133a > 50 % poids, de préférence > 80 %), ces deux composés pouvant être présents en toute proportion relative. Les mélanges à traiter peuvent également contenir jusqu'à 20 % en poids d'autres composés organiques fluorés comme, par exemple, le difluorodichloroéthane (F132b).

Si le mélange initial d'HF et de F133a s'écarte sensiblement de la composition azéotropique, il peut être avantageux d'effectuer au préalable une distillation pour séparer l'azéotrope HF-F133a en tête, du composé en excès (HF ou F133a) qui restera en pied. Après condensation, la composition azéotropique est ensuite soumise à la décantation à froid selon l'invention.

Les exemples suivants illustrent l'invention sans la limiter.

### EXEMPLES 1 à 3

Ces exemples montrent l'intérêt essentiel d'effectuer la séparation du F133a et de l'HF par décantation en présence de TCE (exemples 1 et 2).

Le Tableau I donne la composition de départ des mélanges F133a, HF et TCE et la composition des phases HF et organiques obtenues par décantation à - 20°C.

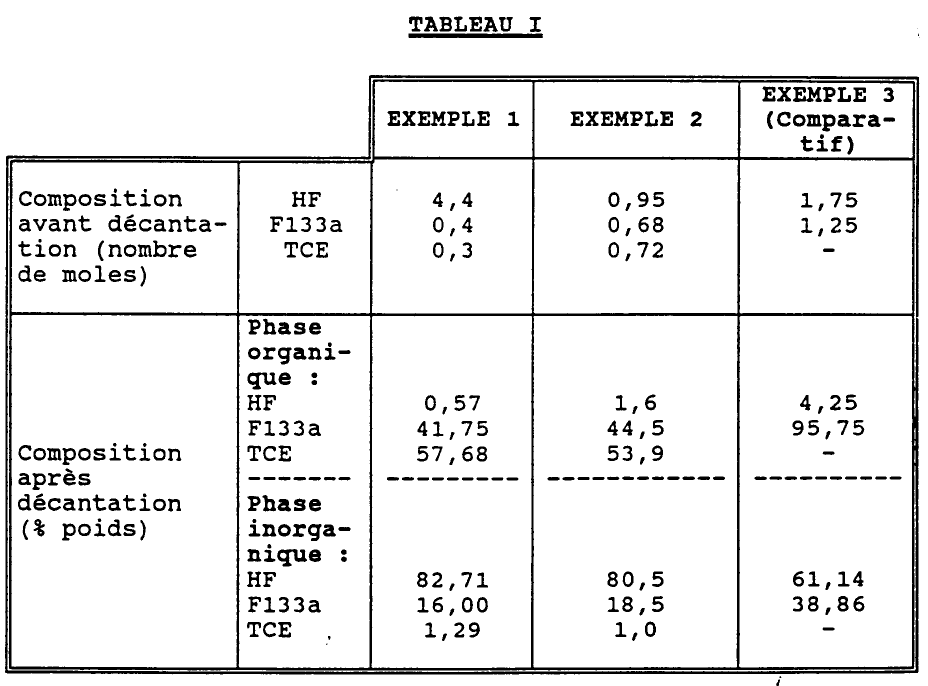

L'examen du tableau I permet de vérifier la nette diminution des solubilités de l'HF dans la phase organique et des organiques dans la phase HF lorsque du TCE est présent.

### EXEMPLE 4

Cet exemple illustre la mise en oeuvre complète du procédé pour l'obtention d'HF, de F133a et de TCE quasi purs, selon la figure 1.

Le mélange de F133a et d'HF initial a la composition suivante : HF 58% mol, F133a 42 % mol. Du TCE est rajouté à ce mélange pour obtenir la composition suivante : HF 40 % mol, F133a 29 % mol, TCE 31 % mol.

Le tableau II donne la composition et les conditions de pression et de température des divers flux par référence à la figure 1.

**TABLEAU II**

| | Phase organique (4) | Phase HF (5) | Tête des colonnes 7 et 11 (9 & 13) | Pied de colonne 7 (8) | Pied de colonne 11 (12) | Tête de colonne 14 (15) | Pied de colonne 14 (16) |
|---|---|---|---|---|---|---|---|
| HF (% moles) | 8,5 | 96,0 | 60 | - | 100 | - | - |
| F133a (%moles) | 41,4 | 3,8 | 40 | 50 | - | 100 | - |
| TCE (% moles) | 50,3 | 0,2 | - | 50 | - | - | 100 |
| Température (°C) | -20 | -20 | 66 | 108 | 96 | 17 | 100 |
| Pression (bars absolus) | 15 | 15 | 10 | 10 | 10 | 1,5 | 1,5 |

## Revendications

1. Procédé de séparation du fluorure d'hydrogène (HF) de ses mélanges avec le 1,1,1-trifluoro-2-chloroéthane (F133a), caractérisé en ce qu'il comprend une étape dans laquelle le mélange HF-F133a à traiter est soumis à une décantation en présence de trichloroéthylène (TCE) à une température inférieure à 0°C pour obtenir une phase supérieure riche en HF et une phase inférieure organique pauvre en HF, le mélange soumis à la décantation contenant de 0,25 à 2,5 moles de TCE par mole de F133a.

2. Procédé selon la revendication 1, dans lequel le mélange soumis à la décantation contient de 0,8 à 1,2 moles de TCE par mole de F133a.

3. Procédé selon la revendication 1 ou 2, dans lequel la température de décantation est comprise entre -40 et -10°C, de préférence entre -25 et -15°C.

4. Procédé selon l'une des revendications 1 à 3, dans lequel, avant l'opération de décantation, le mélange HF-F133a est éventuellement soumis à une distillation pour l'amener a une composition voisine de la composition azéotropique.

5. Procédé selon l'une des revendications 1 à 4, caractérisé en ce que, après décantation :
**a)** la phase inférieure organique pauvre en HF obtenue est distillée de façon à séparer en tête l'HF contenu dans cette phase, sous forme d'azéotrope HF-F133a qui est renvoyé au décanteur et à récupérer en pied un mélange de F133a et de TCE, et
**b)** la phase supérieure riche en HF est soit recyclée directement au réacteur de fluoration, soit soumise à une distillation de façon à séparer en tête le F133a contenu dans cette phase, sous forme d'azéotrope HF-F133a qui est renvoyé au décanteur et à récupérer en pied du HF pratiquement pur.

6. Procédé selon la revendication 5, caractérisé en ce que le mélange de F133a et de TCE est soumis à une distillation de façon à séparer en tête du F133a pur et à récupérer en pied du TCE pur.

7. Procédé selon l'une des revendications 1 à 6, dans lequel le mélange d'HF et de F133a à traiter contient en outre jusqu'à 20 % en poids d'autres composés organiques fluorés.

## Claims

1. Process for the separation of hydrogen fluoride (HF) from its mixtures with 1,1,1-trifluoro-2-chloroethane (F133a), characterised in that it comprises a stage in which the HF/F133a mixture to be treated is subjected to a settling in the presence of trichloroethylene (TCE) at a temperature below 0°C in order to produce an upper phase which is rich in HF and a lower organic phase which is poor in HF, the mixture subjected to settling containing from 0.25 to 2.5 mol of TCE per mole of F133a.

2. Process according to Claim 1, in which the mixture subjected to settling contains from 0.8 to 1.2 mol of TCE per mole of F133a.

3. Process according to Claim 1 or 2, in which the settling temperature is between -40 and -10°C, preferably between -25 and -15°C.

4. Process according to one of Claims 1 to 3, in which, before the settling operation, the HF/F133a mixture is optionally subjected to a distillation in order to bring it to a composition in the region of the azeotropic composition.

5. Process according to one of Claims 1 to 4, characterised in that, after settling:
**a)** the lower organic phase obtained, which is poor in HF, is distilled so as to separate, at the head, the HF contained in this phase, in the form of an HF/F133a azeotrope which is returned to the settler, and to recover, at the foot, a mixture of F133a and TCE, and
**b)** the upper phase, which is rich in HF, is either recycled directly to the fluorination reactor or subjected to a distillation so as to separate, at the head, the F133a contained in this phase, in the form of an HF/F133a azeotrope which is returned to the settler, and to recover, at the foot, practically pure HF.

6. Process according to Claim 5, characterised in that the mixture of F133a and TCE is subjected to a distillation so as to separate, at the head, pure F133a and to recover, at the foot, pure TCE.

7. Process according to one of Claims 1 to 6, in which the mixture of HF and F133a to be treated additionally contains up to 20 % by weight of other fluorinated organic compounds.

## Patentansprüche

1. Verfahren zur Abtrennung von Fluorwasserstoff (HF) aus Mischungen mit 1,1,1-Trifluor-2-chlorethan (F133a), dadurch gekennzeichnet, daß es einen Schritt umfaßt, bei dem die zu behandelnde HF/F133a-Mischung einer Dekantierung in Gegenwart von Trichlorethylen (TCE) bei einer Temperatur unterhalb von 0°C unterworfen wird, um eine HF-reiche obere Phase und eine HF-arme, organische untere Phase zu erhalten, wobei die der Dekantierung unterworfene Mischung 0,25 bis 2,5 mol TCE pro mol F133a enthält.

2. Verfahren nach Anspruch 1, bei dem die der Dekantierung unterworfene Mischung 0,8 bis 1,2 mol TCE pro mol F133a enthält.

3. Verfahren nach Anspruch 1 oder 2, bei dem die Dekantierungstemperatur zwischen -40 und -10°C, vorzugsweise zwischen -25 und -15°C, liegt.

4. Verfahren nach einem der Ansprüche 1 bis 3, bei dem vor der Durchführung der Dekantierung die HF/F133a-Mischung gegebenenfalls einer Destillation unterworfen wird, um eine Zusammensetzung zu erhalten, die etwa der azeotropen Zusammensetzung entspricht.

5. Verfahren nach einem der Ansprüche 1 bis 4, dadurch gekennzeichnet, daß nach der Dekantierung
a) die erhaltene, HF-arme, organische untere Phase destilliert wird, um am Kopf das in dieser Phase enthaltene HF in Form eines HF/133a-Azeotrops abzutrennen, das wieder der Dekantiervorrichtung zugeführt wird, und um am Fuß eine Mischung aus F133a und TCE wiederzugewinnen; und daß
b) die HF-reiche obere Phase entweder dem Fluorierungsreaktor direkt wieder zugeführt wird oder einer Destilllation unterworfen wird, um am Kopf das in dieser Phase enthaltene F133a in Form eines HF/F133a-Azeotrops abzutrennen, das der Dekantiervorrichtung wieder zugeführt wird, und um am Fuß praktisch reines HF wiederzugewinnen.

6. Verfahren nach Anspruch 5 dadurch gekennzeichnet, daß die Mischung aus F133a und TCE einer Destillation unterworfen wird, um am Kopf reines F133a abzutrennen und am Fuß reines TCE wiederzugewinnen.

7. Verfahren nach einem der Ansprüche 1 bis 6, bei dem die zu behandelnde Mischung aus HF und F133a außerdem bis zu 20 Gew.-% weitere fluorierte organische Verbindungen enthält.
